(19) Europäisches Patentamt
European Patent Office
Office européen des brevets

(11) EP 1 201 652 B1

(12) # EUROPEAN PATENT SPECIFICATION

(45) Date of publication and mention of the grant of the patent:
**29.06.2005 Bulletin 2005/26**

(51) Int Cl.7: **C07D 201/08**

(21) Application number: **00309506.4**

(22) Date of filing: **27.10.2000**

(54) **Process for the preparation of lactams**

Verfahren zur Herstellung von Lactamen

Procédé pour la préparation de lactames

(84) Designated Contracting States:
**AT BE CH CY DE DK ES FI FR GB GR IE IT LI LU MC NL PT SE**

(43) Date of publication of application:
**02.05.2002 Bulletin 2002/18**

(73) Proprietor: **Dairen Chemical Corporation**
**Taipei (TW)**

(72) Inventors:
- **Chen, Shien-Chang**
  **Taipei, Taiwan (CN)**
- **Hsu, Liang-An**
  **Kaohsiung, Taiwan (CN)**
- **Tsai, Cheng-Lin**
  **Kaohsiung, Taiwan (CN)**
- **Lin, Joe-Min**
  **Kaohsiung, Taiwan (CN)**
- **Lin, Fu-Shen**
  **Kaohsiung, Taiwan (CN)**

(74) Representative: **Jones, Helen Marjorie Meredith**
**Gill Jennings & Every,**
**Broadgate House,**
**7 Eldon Street**
**London EC2M 7LH (GB)**

(56) References cited:
**DE-B- 1 148 553**
**FR-A- 2 102 606**
**US-A- 2 882 243**
**US-A- 2 882 244**
**US-A- 3 130 007**
**US-A- 3 775 431**
**US-A- 3 888 845**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the Invention

The present invention is related to a method for preparing lactam, which comprises an amination reaction using crystalline aluminosilicate zeolites as catalysts under the condition of gas phase in the presence of (a) lactone, (b) amine and/or ammonia and (c) water. The method of the present invention, which uses crystalline aluminosilicate zeolite catalysts, has many advantages including low reaction pressure, high yield per unit time, and short reaction time, etc.

### Background of the Invention

Lactam, such as 2-pyrrolidone, N-alkylpyrrolidone, caprolactam etc., is used as a solvent or a polymeric monomer of high molecular compounds. Thus, developing an economic method for preparing lactam is a common industrial requirement.

For example, pyrrolidone may be synthesized by a hydrogenation and an amination reaction with succinic acid, succinic acid anhydrate, maleic acid or maleic acid anhydrate, etc.; caprolactam may be synthesized by a hydrogenation and an amination reaction with cyclohexanone. Moreover, lactam may also be synthesized by an amination of lactone and amine with a method of catalytic reaction or a method of non-catalytic reaction.

Regarding the method of non-catalytic liquid phase reaction, Japanese Patent Application Nos. Sho-47-21420 and Sho-49-20585 disclose a method of reacting an excessive amount of aqueous methylamine solution with γ-butyrolactone and generating N-methylpyrrolidone at a temperature of 200 to 300 under 20 to 40 atm. In this method, methylamine is first dissolved in the water, and then reacted with γ-butyrolactone, which is effective for the selectivity of N-methylpyrrolidone. Japanese Patent Application No. Sho-51-42107 discloses a method of dissolving an excessive amount of methylamine in the water, and recycling methylamine in the reaction by water carrying methylamine. Japanese Patent Examined Publication Nos. Hei-6-78304 and Hei-7-10835 disclose an improved method of using secondary or tertiary amine for preparing N-substituted-2-pyrrolidone. However, such non-catalytic liquid phase reactions described above still have many disadvantages, including highpressure operation and low yield per unit time (i.e., long reaction time), so that it does not yield a beneficial economic effect in the industrial process. Furthermore, in these reactions, industry needs to use large reactor equipment because of the high reaction pressure and the long retention time of the product in the reactor, so that its manufacturing cost is prohibitive.

Regarding the method of catalytic liquid phase reaction, Paul G. Rodewald *et al.* first proposed in USP 3,775,431 in 1973 a process by which lactone is reacted with primary amine and generates lactam using Zeolite X as catalysts. However, for example, in generating N-methylpyrrolidone, the yield of resultant products is still poor although the reaction temperature reaches 300°C, the reaction pressure reaches 500 psig and an excessive amount of methylamine is used, such as where the molar ratio is over 30.

We, the inventors, have broadly and deeply studied the defects of the traditional technique, and found that an amination reaction of lactone and amine and/or ammonia using crystalline aluminosilicate zeolites as catalysts under the condition of gas phase may substantially decrease the reaction pressure of operation and increase the yield of products. We have hereby accomplished the present invention.

### Detailed Description of the Invention

The present invention is related to a method for preparing lactam represented by the following formula (I):

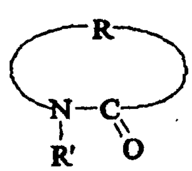

(I)

wherein R is $C_{2-10}$ alkylene which may be optionally substituted with $C_{1-6}$ alkyl or phenyl; R' is a hydrogen atom, $C_{1-6}$ alkyl, $C_{1-6}$ hydroxyalkyl or phenyl. The method for preparing lactam comprises an amination reaction using crystalline aluminosilicate zeolites as catalysts under the condition of gas phase in the presence of (a) lactone, (b) amine and/or ammonia and (c) water.

Lactone, the starting material used in the present invention, may be represented by the following formula (II):

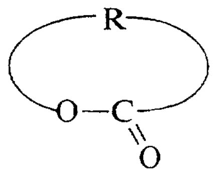

(II)

wherein the definition of R has the same meaning described above.

In the present invention, the example of $C_{2-10}$ alkylene represented by R includes ethylene, propylene, butylene, pentylene, hexylene, heptylene, octylene, nonylene, decylene, etc. $C_{2-10}$ alkylene represented by R may be optionally substituted with $C_{1-6}$ alkyl or phenyl. The example of $C_{1-6}$ alkyl as a substituent includes methyl, ethyl, propyl, butyl, pentyl, hexyl, etc.

The preferred example of lactone used in the present invention includes β-propiolactone, γ-butyrolactone, γ-phenyl-γ-butyrolactone, γ-methyl-γ-butyrolactone, γ-phenyl-γ-methyl-γ-butyrolactone, δ-butyrolactone, γ-valerolactone, γ-caprolactone, ε-caprolactone, δ-hydroxyoctylic acid lactone (δ-(hydroxyoctanoic acid lactone), δ-hydroxynonylic acid lactone (δ-hydroxynonanoic acid lactone), δ-hydroxydecylic acid lactone (δ-hydroxydecanoicacid lactone), etc., more preferably γ-butyrolactone, γ-caprolactone and ε-caprolactone.

Amine, the starting material used in the present invention, may be primary, secondary or tertiary acyclic amine substituted with one to three $C_{1-6}$ alkyl, $C_{1-6}$ hydroxyalkyl or phenyl. While mono-, di- or tri-$C_{1-6}$ alkylamine, mono-, di- or tri- $C_{1-6}$ alkanolamine is preferred, the example includes mono-, di- or trimethylamine, mono-, di- or tri-ethylamine, n-propylamine, n-butylamine, n-hexylamine, mono-, di- or tri-ethanolamine, etc.

The present invention utilizes crystalline aluminosilicate zeolites as catalysts for the amination reaction. The crystalline aluminosilicate zeolite has an excellent reactive effect in comparison with other conventional zeolite catalysts, such as mordenite ($Na_8Al_8Si_{40}O_{96}$ $24H_2O$), Y-type zeolite, etc. In the crystalline aluminosilicate zeolite, silicon dioxide and aluminum oxide are in the ratio of (30 to 500): 1, and the constraint index is 1 to 12. The preferred example of the crystalline aluminosilicate zeolite includes ZSM-5, ZSM-11, ZSM-12, ZSM-22, ZSM-34, ZSM-35, ZSM-48, etc. ZSM-5 catalysts with three dimensional structures in which the pore size is from 5 to 6 angstrom between small pore molecular sieves (such as A-type zeolite, calcium magnesium zeolite, etc.) and large pore molecular sieves (such as octahedral zeolite, mordenite, etc.) is the most preferred. Therefore, the amination of the present invention is a heterogeneous reaction.

Lactone and amine and/or ammonia used in the amination reaction of the present invention must mix with water in a proper ratio in advance to use as the reactant. In the reactant, the amount of amine and/or ammonia may be more or less than the amount of lactone; in general, an excessive amount of amine and/or ammonia is preferably used.

The molar ratio of lactone and amine and/or ammonia used in the present invention usually ranges from (1:0.5) to (1:30), preferably from (1:1) to (1:10). The increase of the molar ratio of feeding lactone and amine and/or ammonia, such as the increase of 1:5 or more, may shorten the reaction time, as well as easily purify and separate the excessive amount of amine and/or ammonia from the product after the reaction. However, if the molar ratio is over the upper limit, the selectivity may become poor. If the molar ratio is below the lower limit, amine and/or ammonia become the limiting reagent, so that it is difficult to purify and separate the excessive amount of lactone from the product after the reaction.

The molar ratio of lactone and water used in the present invention usually ranges from (1:0.5) to (1:20), preferably from (1:2) to (1:6). If the molar ratio is below the lower limit, the conversion and selectivity of the reaction may obviously become poor. If the molar ratio is over the upper limit, the purification and separation of the product after the reaction may cost excessive amounts of time and energy, although the reaction is not directly affected.

The temperature of the amination reaction used in the present invention ranges from 180 to 400°C , preferably 220 to 320°C . In general, the selectivity of the reaction may be raised with the increase of the temperature. However, if the temperature is over 400°C , undesired by-products may be yielded in addition to the resultant lactam. If the temperature is below 180°C , the reaction may not be carried out rapidly enough to be effective.

Because the acidity of the crystalline aluminosilicate zeolite described above is stronger than aluminum oxide and the reactivity is also strong, the amination reaction of the present invention may be carried out at a pressure which does not destroy the configuration of the catalyst. The pressure used in the reaction usually ranges from 0 to 10 atm., preferably from 1 to 5 atm. While the increase of the pressure may slightly raise the conversion of lactone, the effect is not significant in comparison with other factors affecting the experiment.

The amination reaction of the present invention may be carried out in the fixed bed reactor, the fluid bed reactor, and the other heterogeneous gas phase reactor. If the fixed bed reactor is used, the crystalline aluminosilicate zeolite catalyst described above needs to form shapes for easy packing, such as in grains, tablets, etc.

The gas hourly space velocity (hereinafter sometimes abbreviated as GHSV) used in the amination reaction

of the present invention may depend on the reaction of various lactone and amine and/or ammonia. In general, GHSV is 20 to 100000 $hr^{-1}$, preferably 2000 to 50000 $hr^{-1}$. If GHSV is over 100000 $hr^{-1}$, the reaction is not completed, and the conversion is poor. If GHSV is below 20 $hr^{-1}$, undesired by-products may easily result due to the lengthy time of contacting with the catalyst.

The present invention will be further described in the following Examples. However, such Examples are merely used to specify the present invention, and they are not used to restrict the scope of the present invention.

**Example**

Lactone and amine and/or ammonia used in the following Example and Reference Example were well-known compounds or prepared by the conventional methods.

$$\text{The selectivity of Lactam (\%)} = \frac{\text{The mole of Lactam Product}}{\text{The Mole of Feed-in Lactone - The Mole of feed-out Lactone}} \times 100 \qquad (2)$$

$$\text{The conversion of Lactone (\%)} \quad \frac{\text{The Mole of Feed-in Lactone - The Mole of Feed-out Lactone}}{\text{The Mole of Feed-in Lactone}} \times 100\% \qquad (1)$$

In a given time after the reaction, the product was collected by condensation. The component of the efflux from the outlet was analyzed by HP-6890 gas chromatograph. The conversion of lactone and the selectivity of lactam were calculated according to the following equation (1) and (2):

Example 1

ZSM-5 catalyst (40 ml) was packed in the reactor having an inside diameter of 26 mm. The reactants of γ-butyrolactone, methylamine and water were mixed and fed in the ratio of 10: 4: 6 by weight. The gas hourly space velocity of feeding the mixed reactants was maintained 5050 $hr^{-1}$, and the amination reaction was carried out at 280°C . The product was then collected and analyzed. The conversion of γ-butyrolactone was 99.4%, and the selectivity of N-methyl-2-pyrrolidone was 99.0%.

Reference Example 1

The reactor and catalyst were used the same way as in Example 1, but the reactants were only γ-butyrolactone and methylamine which were mixed and fed in the ratio of 1: 18 by weight. The gas hourly space velocity of feeding the mixed reactants was maintained 80000 $hr^{-1}$, and the amination reaction was carried out at 280°C. The product was then collected and analyzed. The conversion of γ-butyrolactone was 35.0%, and the selectivity of N-methyl-2-pyrrolidone was 50.0%.

Example 2

The preparation conditions including the reactor, the catalyst, GHSV and the reaction temperature were all the same as in Example 1 except that the reactants of γ-butyrolactone, methylamine and water were mixed and fed in the ratio of 10: 4: 6 by weight. The product was then collected and analyzed. The conversion of γ-butyrolactone was 99.7%, and the selectivity of N-methyl-2-pyrrolidone was 99.9%.

Example 3 to 8

The preparation conditions including the reactor, the ratio of feeding the reactants, GHSV and the reaction temperature were all the same as in Example 2 except that various series of ZSM catalysts were used. The product was collected and analyzed. The results are shown in Table 1.

Table 1

| Example | Catalyst Type | The Conversion of Butyrolactone | The Selectivity of N-methyl-2-pyrrolidone |
|---|---|---|---|
| Example 2 | ZSM-5 | 99.7% | 99.9% |
| Example 3 | ZSM-11 | 98.0% | 83.7% |
| Example 4 | ZSM-12 | 97.6% | 81.3% |
| Example 5 | ZSM-22 | 98.2% | 78.0% |
| Example 6 | ZSM-34 | 96.6% | 75.0% |
| Example 7 | ZSM-35 | 97.9% | 69.3% |
| Example 8 | ZSM-48 | 98.1% | 64.4% |

Example 9 to 11

The preparation conditions including the reactor, the catalyst, the ratio of feeding the reactants and the reaction temperature were all the same as in Example 2 except that various GHSV were used. The product was collected and analyzed after stabilization. The results are shown in Table 2.

Table 2

| Example | GHSV($hr^{-1}$) | The Conversion of Butyrolactone | The Selectivity of N-methyl-2-pyrrolidone |
|---|---|---|---|
| Example 2 | 5050 | 99.7% | 99.9% |
| Example 9 | 7200 | 99.3% | 93.7% |
| Example 10 | 3000 | 100% | 99.9% |
| Example 11 | 1000 | 100% | 97.5% |

The preparation conditions including the reactor, the ratio of feeding the reactants, GHSV and the reaction temperature were all the same as in Example 2 except that mordenite (40 ml) was used as the catalyst. The product was then collected and analyzed. The conversion of γ-butyrolactone was 99.5%, and the selectivity of N-methyl-2-pyrrolidone was 58.0%.

Example 12

The reactor and catalyst were used the same way as in Example 1, but the reactants of γ-butyrolactone and water were mixed and fed in the ratio of 1.2: 1 by weight, and ammonia gas was continuously feed-in by using a flow controller at the same time. The gas hourly space velocity of feeding the mixed reactants was maintained 3300 $hr^{-1}$, and the amination reaction was carried out at 300°C. The product was then collected and analyzed. The conversion of γ-butyrolactone was 96.0%, and the selectivity of 2-pyrrolidone was 90.0%.

Reference Example 3

The reactor and catalyst were used the same way as in Example 1, but the reactants were only γ-butyrolactone and ammonia gas which were mixed and fed in the ratio of 1: 2 by weight. The gas hourly space velocity of feeding the mixed reactants was maintained 2700 $hr^{-1}$, and the amination reaction was carried out at 300°C. The product was then collected and analyzed. The conversion of γ-butyrolactone was 20.0%, and the selectivity of 2-pyrrolidone was 65.0%.

Reference Example 4

The reactor, the ratio of feeding the reactants and the reaction temperature were all the same as in Example 12. Mordenite (40 ml) was used as the catalyst, and the gas hourly space velocity of feeding the mixed reactants was maintained 2700 $hr^{-1}$. The product was then collected and analyzed. The conversion of γ-butyrolactone was 80.0%, and the selectivity of 2-pyrrolidone was 40.0%.

Example 13

The reactor and catalyst were used the same way as in Example 1, but the reactants of γ-butyrolactone, dimethylamine and water were mixed and fed in the ratio of 10: 7: 11 by weight, and the reactants were continuously feed-in by using a flow controller. The gas hourly space velocity of feeding the mixed reactants was maintained 1000 $hr^{-1}$, and the amination reaction was carried out at 280 . When the product was collected and analyzed, 10% of methanol was in the product. The conversion of γ-butyrolactone was 98.0%, and the selectivity of N-methyl-2-pyrrolidone was 88.0%.

Example 14

The preparation conditions including the reactor, the catalyst, GHSV and the reaction temperature were all the same as in Example 13 except that the reactants of γ-butyrolactone, trimethylamine and water were mixed and fed in the ratio of 10: 9.3: 11 by weight. When the product was collected and analyzed, 8% of methanol was in the product. The conversion of γ-butyrolactone was 95.0%, and the selectivity of N-methyl-2-pyrrolidone was 79.0%.

Reference Example 5

The preparation conditions including the reactor, the ratio of feeding the reactants, GHSV and the reaction temperature were all the same as in Example 13 except that Y-type zeolite (40 ml) was used as the catalyst. When the product was collected and analyzed, 6.6% of methanol was in the product. The conversion of γ-butyrolactone was 95.0%, and the selectivity of N-methyl-2-pyrrolidone was 51.0%.

Example 15

The preparation conditions including the reactor, the catalyst, GHSV and the reaction temperature were all the same as in Example 13 except that the reactants of γ-butyrolactone, ethylamine and water were mixed and fed in the ratio of 10: 7: 13.6 by weight. The product was then collected and analyzed. The conversion of γ-butyrolactone was 98.0%, and the selectivity of N-ethyl-2-pyrrolidone was 79.0%.

Example 16

The preparation conditions including the reactor, the catalyst, GHSV and the reaction temperature were all the same as in Example 13 except that the reactants of γ-butyrolactone, triethylamine and water were mixed and fed in the ratio of 10: 16.4: 14.6 by weight. When the product was collected and analyzed, 13% of ethanol was in the product. The conversion of γ-butyrolactone was 95.0%, and the selectivity of N-ethyl-2-pyrrolidone was 68.0%.

Example 17

The preparation conditions including the reactor, the catalyst, GHSV and the reaction temperature were all the same as in Example 13 except that the reactants of γ-butyrolactone, ethanolamine and water were mixed and fed in the ratio of 7: 5: 6 by weight. The product was then collected and analyzed. The conversion of γ-butyrolactone was 92.0%, and the selectivity of N-(β-ethyl)-2-pyrrolidone was 69.0%.

According to the above Examples and Reference Examples, the conversion and selectivity of the reaction were found to be very poor when the ratio of water in the reactants was too low or even non-existent (i.e., Reference Examples 1 and 3); thus it was demonstrated that water was a necessary reactant in the present invention. Since the selectivity of lactam was not raised when the conventional mordenite or Y-type zeolite was used as the catalyst (i.e., Reference Examples 2, 4 and 5), it was demonstrated that the crystalline aluminosilicate zeolite of the present invention raised the yield per unit time.

In addition, compared with the conventional technique, the conditions of generating lactam vary with the kinds of amine utilized when non-catalytic liquid phase amination reaction is used; however, all of the reactions had to be carried out at a high pressure. Using the crystalline aluminosilicate zeolite of the present invention may rapidly obtain the desired lactam at a low pressure.

It is understood that variations and modifications can be effected without departing from the spirit or scope of the invention as set forth in the following claims.

## Claims

1. A method for preparing lactam represented by the following formula (I):

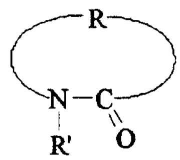

(I)

wherein R is $C_{2-10}$ alkylene which may be optionally substituted with $C_{1-6}$ alkyl or phenyl; R' is a hydrogen atom, $C_{1-6}$ alkyl, $C_{1-6}$ hydroxyalkyl or phenyl; which comprises an amination reaction using crystalline aluminosilicate zeolites as catalysts under the condition of gas phase in the presence of (a) lactone represented by the following formula (II):

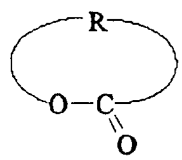

(II)

wherein the definition of R has the same meaning as described above;
wherein said amine is a primary, secondary or tertiary acyclic amine substituted with one to three $C_{1-6}$ alkyl, $C_{1-6}$ hydroxyalkyl or phenyl b) amine and/or ammonia **characterized in that** the amination reaction is carried out in the presence of water.

2. The method of claim 1, wherein said lactone is isβ-propiolactone, γ-butyrolactone, δ-butyrolactone, γ-valerolactone, γ-caprolactone, ε-caprolactone, δ-hydroxyoctylic acid lactone, δ-hydroxynonylic acid lactone, or δ-hydroxydecylic acid lactone.

3. The method of claim 1, wherein said amine is mono-, di- or tri-$C_{1-6}$ alkylamine, mono-, di- or tri- $C_{1-6}$ alkanolamine.

4. The method of claim 3, wherein said amine is mono-, di- or tri-methylamine, mono-, di- or tri-ethylamine, mono-, di- or tri-ethanolamine.

5. The method of claim 1, wherein the molar ratio of silicon dioxide and aluminum oxide in said crystalline aluminosilicate zeolite is (30 to 500): 1.

6. The method of claim 1, wherein the constraint index of said crystalline aluminosilicate zeolite is 1 to 12.

7. The method of any one of claim 1 to claim 6, wherein said crystalline aluminosilicate zeolite is selected from a group consisting of ZSM-5, ZSM-11, ZSM-12, ZSM-22, ZSM-34, ZSM-35 and ZSM-48.

8. The method of claim 7, wherein said crystalline aluminosilicate zeolite is ZSM-5.

9. The method of claim 1, wherein the molar ratio of feeding said lactone and said amine and/or ammonia ranges from (1:0.5) to (1:30).

10. The method of claim 1, wherein the molar ratio of feeding said lactone and said water ranges from (1:0.5) to (1:20).

11. The method of claim 1, wherein the temperature of said amination reaction ranges from 180 to 400°C.

12. The method of claim 1, wherein the pressure of said amination reaction ranges from 0 to 10 atm.

13. The method of claim 1, wherein the gas hourly space velocity (GHSV) of said amination reaction is 20 to 100000

$hr^{-1}$.

**14.** The method of claim 1, wherein the amination reaction is carried out in the fixed bed reactor or fluid bed reactor.

**Patentansprüche**

**1.** Verfahren zur Herstellung eines Lactams gemäß folgender Formel (I):

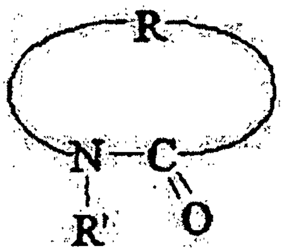

(I)

worin R ein $C_{2-10}$-Alkylen, welches gegebenenfalls mit einem $C_{1-6}$-Alkyl oder Phenyl substituiert sein kann, ist, und R' ein Wasserstoffatom, $C_{1-6}$-Alkyl, $C_{1-6}$-Hydroxyalkyl oder Phenyl ist, welches eine Aminierungsreaktion unter Verwendung von kristallinen Aluminiumsilikatzeolithen als Katalysatoren in einer Gasphase in Gegenwart (a) eines Lactons gemäß folgender Formel (II):

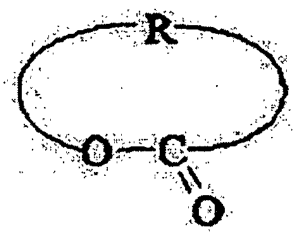

(II)

worin R wie oben definiert ist, und (b) eines Amins und/oder Ammoniak, wobei das Amin ein primäres, sekundäres oder tertiäres azyklisches Amin ist, welches mit einem bis drei $C_{1-6}$-Alkyl, $C_{1-6}$-Hydroxyalkyl oder Phenyl substituiert ist, umfasst, **dadurch gekennzeichnet, dass** die Aminierungsreaktion in Gegenwart von Wasser durchgeführt wird.

**2.** Verfahren gemäß Anspruch 1, wobei es sich bei dem Lacton um β-Propiolacton, γ-Butyrolacton, δ-Butyrolacton, γ-Valerolacton, γ-Caprolacton, ε-Caprolacton, δ-Hydroxyoctylsäurelacton, δ-Hydroxynonylsäurelacton oder δ-Hydroxydecylsäurelacton handelt.

**3.** Verfahren gemäß Anspruch 1, wobei das Amin ein Mono-, Di- oder Tri-$C_{1-6}$-Alkylamin, oder Mono-, Di- oder Tri-$C_{1-6}$-Alkanolamin ist.

**4.** Verfahren gemäß Anspruch 3, wobei das Amin ein Mono-, Di- oder Trimethylamin, Mono-, Di- oder Triethylamin, oder Mono-, Di- oder Triethanolamin ist.

**5.** Verfahren gemäß Anspruch 1, wobei das molare Verhältnis des Siliziumdioxids zu dem Aluminiumoxid in dem kristallinen Aluminiumsilikatzeolithen 30-500:1 beträgt.

**6.** Verfahren gemäß Anspruch 1, wobei der Zwangsindex des kristallinen Aluminiumsilikatzeolithen 1 bis 12 beträgt.

**7.** Verfahren gemäß wenigstens einem der Ansprüche 1 bis 6, wobei es sich bei dem kristallinen Aluminiumsilikatzeolithen um ZSM-5, ZSM-11, ZSM-12, ZSM-22, ZSM-34, ZSM-35 und/oder ZSM-48 handelt.

**8.** Verfahren gemäß Anspruch 7, wobei der kristalline Aluminiumsilikatzeolith ZSM-5 ist.

**9.** Verfahren gemäß Anspruch 1, wobei das molare Einspeisungsverhältnis des Lactons zu dem Amin und/oder Ammoniak im Bereich von 1:0,5 bis 1:30 liegt.

**10.** Verfahren gemäß Anspruch 1, wobei das molare Einspeisungsverhältnis des Lactons zu dem Wasser im Bereich von 1:0,5 bis 1:20 liegt.

**11.** Verfahren gemäß Anspruch 1, wobei die Temperatur der Aminierungsreaktion im Bereich von 180 bis 400 °C liegt.

**12.** Verfahren gemäß Anspruch 1, wobei der Druck der Aminierungsreaktion im Bereich von 0 bis 10 atm liegt.

**13.** Verfahren gemäß Anspruch 1, wobei die stündliche Raumgeschwindigkeit des Gases (GHSV) bei der Aminierungsreaktion 20 bis 100.000 $Std.^{-1}$ beträgt.

**14.** Verfahren gemäß Anspruch 1, wobei die Aminierungsreaktion in einem Festbettreaktor oder Fließbettreaktor durchgeführt wird.

**Revendications**

**1.** Procédé de préparation de lactame représenté par la formule (I) suivante :

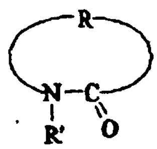

(I)

dans laquelle R représente un alkylène en $C_2$ à $C_{10}$ qui peut le cas échéant être substitué par un radical alkyle en $C_1$ à $C_6$ ou phényle, R représente un atome d'hydrogène, un radical alkyle en $C_1$ à $C_6$, hydroxyalkyle en $C_1$ à $C_6$ ou phényle, lequel procédé comprend une réaction d'amination utilisant comme catalyseurs des zéolithes d'aluminosilicates cristallins, en condition de phase gazeuse, en présence de (a) une lactone représentée par la formule (II) suivante :

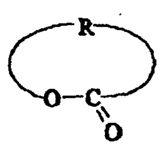

(II)

dans laquelle la définition de R a la même signification que celle décrite ci-avant,
où ladite amine est une amine primaire, secondaire ou tertiaire acyclique substituée par un à trois radicaux alkyle en $C_1$ à $C_6$, hydroxyalkyle en $C_1$ à $C_6$ ou phényle, (b) d'une amine et/ou d'ammoniac, **caractérisé en ce que** la réaction d'amination est entreprise en présence d'eau.

**2.** Procédé selon la revendication 1, dans lequel ladite lactone est de la β-propiolactone, de la γ-butyrolactone, de la δ-butyrolactone, de la γ-valérolactone, de la γ-caprolactone, de l'ε-caprolactone, de la lactone d'acide δ-hydroxyoctylique, de la lactone d'acide δ-hydroxynonylique ou de la lactone d'acide δ-hydroxydécylique.

**3.** Procédé selon la revendication 1, dans lequel ladite amine est une mono-, di- ou trialkylamine en $C_1$ à $C_6$, une mono-, di- ou trialcanolamine en $C_1$ à $C_6$.

**4.** Procédé selon la revendication 3, dans lequel ladite amine est de la mono-, di- ou triméthylamine, de la mono-, di- ou triéthylamine, de la mono-, di- ou triéthanolamine.

**5.** Procédé selon la revendication 1, dans lequel le rapport molaire du dioxyde de silicium et de l'oxyde d'aluminium

dans ladite zéolithe d'aluminosilicate cristallin est de (30 à 500) : 1.

**6.** Procédé selon la revendication 1, dans lequel l'indice de contrainte de ladite zéolithe d'aluminosilicate cristallin est de 1 à 12.

**7.** Procédé selon l'une quelconque des revendications 1 à 6, dans lequel ladite zéolithe d'aluminosilicate cristallin est choisie parmi un groupe formé par les ZSM-5, ZSM-11, ZSM-12, ZSM-22, ZSM-34, ZSM-35 et ZSM-48.

**8.** Procédé selon la revendication 7, dans lequel ladite zéolithe d'aluminosilicate cristallin est de la ZSM-5.

**9.** Procédé selon la revendication 1, dans lequel le rapport molaire d'alimentation de ladite lactone et de ladite amine et/ou d'ammoniac est de (1 : 0,5 ) à (1 : 30).

**10.** Procédé selon la revendication 1, dans lequel le rapport molaire d'alimentation de ladite lactone et de ladite eau est de (1 : 0,5) à (1 : 20).

**11.** Procédé selon la revendication 1, dans lequel la température de ladite réaction d'amination est de 180 à 400°C.

**12.** Procédé selon la revendication 1, dans lequel la pression de la dite réaction d'amination est de 0 à 10 atm.

**13.** Procédé selon la revendication 1, dans lequel la vitesse spatiale horaire de gaz (GHSV) de ladite réaction d'amination est de 20 à 100000 $h^{-1}$.

**14.** Procédé selon la revendication 1, dans lequel la réaction d'amination est entreprise dans un réacteur à lit fixe ou un réacteur à lit fluide.